## Europäisches Patentamt

## European Patent Office

## Office européen des brevets

(11) Veröffentlichungsnummer: **0 175 830**
**A1**

(12) # EUROPÄISCHE PATENTANMELDUNG

(21) Anmeldenummer: **84810468.3**

(22) Anmeldetag: **26.09.84**

(51) Int. Cl.⁴: **A 61 M 1/00**

(43) Veröffentlichungstag der Anmeldung:
**02.04.86 Patentblatt 86/14**

(84) Benannte Vertragsstaaten:
**AT BE CH DE FR GB IT LI LU NL SE**

(71) Anmelder: **Bay, Otto, Dipl. Masch. Ing.**
**Luzernstrasse 45**
**CH-4553 Subingen(CH)**

(72) Erfinder: **Bay, Otto, Dipl. Masch. Ing.**
**Luzernstrasse 45**
**CH-4553 Subingen(CH)**

(72) Erfinder: **Hauri, Hermann**
**Fliederweg 6**
**CH-5600 Lenzburg(CH)**

(74) Vertreter: **Eder, Carl E. et al,**
**Patentanwaltsbüro EDER AG Münchensteinerstrasse 2**
**CH-4052 Basel(CH)**

(54) **Saugvorrichtung für die Wunddrainage.**

(57) Die Saugvorrichtung (1) weist ein Sauggefäss (11) mit einer Vakuum-Anzeigevorrichtung (15) auf. In einem durchsichtigen, mit einer Skala versehenen, an die Decke des Sauggefässes (11) angeformten Stutzen (11a) ist eine Feder (25) eingesetzt, die mit ihrem dem Sauggefäss-In-nenraum zugewandten Ende auf einer Auflagefläche (11b) aufliegt. Ein Mess-und Anzeigeorgan (27) besitzt einen im Stutzen (11a) angeordneten, Falten aufweisenden Balg (27a), dessen In-nenraum am dem Sauggefäss-Innenraum zugewandten Balg-Ende durch einen Anzeigeteil (27b) dicht abgeschlossen ist, der von der Feder (25) mit einer Druckkraft beaufschlagt wird. Beim anderen Ende des Balgs (27a) hängt dieser mit einem Kragen (27c) zusammen, der mit einer Klemmhülse (29) am Stutzen (11a) festgeklemmt ist, wobei der Innenraum des Balgs (27a) bei diesem Ende fluidmässig mit der Umgebung verbunden ist. Wenn der im Innenraum des Sauggefässes (11) herrschende Unterdruck bei der Benutzung der Saugvorrichtung (1) abnimmt, verschiebt die Feder (25) den Anzeigeteil (27b) vom Sauggefäss-Innenraum weg, was eine relativ genaue und gut ablesbare Anzeige des Unterdrucks ergibt.

./...

Croydon Printing Company Ltd.

Fig. 2

Otto Bay, Subingen (Schweiz)


Saugvorrichtung für die Wunddrainage

_____


Die Erfindung betrifft eine Saugvorrichtung gemäss dem Oberbegriff des Anspruchs 1.

Derartige Saugvorrichtungen werden in der Medizin verwendet, um Wundsekrete aus Wunden, insbesondere Oparationswunden, abzusaugen. Dabei wird eine Drainageleitung in die Wunde eingezogen und mit einem vorgängig evakuierten Sauggefäss verbunden. Wenn Wundsekret in das Sauggefäss hineingesaugt wird, steigt der Druck in diesem gegen den in der Umgebung herrschenden Luftdruck an, wodurch die Saugwirkung abnimmt. Wenn die Druckdifferenz zwischen dem Umgebungs-Luftdruck und dem Druck im Innenraum des Sauggefässes zu klein geworden ist, um eine ausreichende Saugwirkung zu erzielen, sollte das Sauggefäss durch ein anderes Sauggefäss ersetzt werden.

Aus der deutschen Gebrauchsmusterschrift 73 36 232 ist eine Saugvorrichtung gemäss dem Oberbegriff des Anspruchs 1 bekannt, die ein mit einem Verschlussteil verschliessbares, für die mehrmalige Verwendung vorgesehenes Sauggefäss und eine Vakuum-Anzeigevorrichtung aufweist. Diese besitzt einen Faltenbalg, dessen eines Ende durch einen zylindrischen, stutzenartigen Teil mechanisch mit dem Verschlussteil des Sauggefässes und

Zb/mm/16497/Fall 4

dessen Innenraum fluidmässig mit dem Innenraum des Sauggefässes verbunden sind. Das dem Verschlussteil abgewandte Ende des Faltenbalgs ist durch eine Abschlusswand abgeschlossen und entlang der Längsachse des Faltenbalgs bewegbar, so dass es seine Lage in Abhängigkeit von der Grösse des Druckes im Sauggefäss- Innenraum ändert und also einen Anzeigeteil bildet. Das Material des Faltenbalgs ist in der Gebrauchsmusterschrift nicht angegeben, wobei man jedoch vermuten mag, dass der Faltenbalg und der diesen mit dem Verschlussteil verbindende, zylindrische, stutzenartige Teil zusammen aus einem einstückigen Körper aus Gummi bestehen.

Bei dieser Vakuum-Anzeigevorrichtung greift an der den Faltenbalg abschliessenden Abschlusswand einerseits eine von der Grösse der Druckdifferenz zwischen der Umgebung und dem Sauggefäss-Innenraum angreifende Fluiddruckkraft und anderseits eine dieser entgegenwirkende Rückstellkraft an, die durch die elastische Deformation des Faltenbalgs bewirkt wird. Die sich bei einer bestimmten Druckdifferenz ergebende Lage der Abschlusswand hängt also stark von den elastischen Eigenschaften des Faltenbalgs, nämlich von der Federkonstante des von diesem gebildeten, elastischen Glieds ab. Da ein Faltenbalg eine verhältnismässig komplizierte Form hat und da der Elastizitätsmodul einer bestimmten Gummiart wegen kleiner Materialunterschiede sowie Temperatur- und Alterungseinflüssen ziemlich stark variieren kann, können die Federkonstanten der einzelnen Faltenbälge bei der serienmässigen Herstellung von solchen in einem verhältnismässig grossen Bereich variieren. Dementsprechend können bei in grossen Stückzahlen hergestellten Saugvorrichtungen auch die sich bei einer bestimmten Druckdifferenz zwischen der Umgebung und dem Sauggefäss-Innenraum ergebenden Lagen der genannten Abschlusswand bezüglich des Verschlussteils stark streuen. Dazu kommt noch, dass bei der Vakuum-Anzeigevorrichtung gemäss der deutschen Gebrauchsmusterschrift 73 36 232 keine Skala vorhanden ist und dass sich auch kein bezüglich des Sauggefässes fester Teil so nahe bei der genannten Abschlusswand befindet, dass er eine Person beim Betrachten des Faltenbalges als Bezugsstelle für die Ermittlung der Lage der Abschlusswand dienen könnte. Die bei der Saugvorrichtung gemäss der Deutschen Gebrauchsmusterschrift 73 36 232 bekannte Vakuum-Anzeigevorrichtung ermöglicht also nur eine sehr ungenaue Ermittlung der Grösse des Unterdrucks im Sauggefäss- Innenraum, d.h. der Druckdifferenz zwischen der Umgebung und dem Sauggefäss-Innenraum. Es ist daher für eine an einer Operation und/oder Pflege eines Patienten beteiligte Person verhältnismässig schwierig festzustellen, wann die genannte Druckdifferenz so niedrig geworden ist, dass das Sauggefäss ersetzt werden sollte.

Die Erfindung will nun hier Abhilfe schaffen und eine Saugvorrichtung bereitstellen, bei der die zwischen der Umgebung und dem Innenraum des Sauggefässes vorhandene Druckdifferenz ausreichend genau ermittelt werden kann, um bei der Benutzung eines Sauggefässes mühelos entscheiden zu können, wann das letztere ersetzt werden muss. Dabei soll die Vakuum-Anzeigevorrichtung kostengünstig herstellbar und derart beschaffen sein, dass sie insbesondere auch bei einem nur für einen einmaligen Gebrauch bestimmten Sauggefäss vorgesehen werden kann.

Diese Aufgabe wird durch eine Saugvorrichtung der einleitend genannten Art gelöst, wobei die Saugvorrichtung nach der Erfindung durch den kennzeichnenden Teil des Anspruchs 1 gekennzeichnet ist. Vorteilhafte Ausgestaltungen der Erfindung ergeben sich aus den abhängigen Ansprüchen.

Die Erfindung wird nachstehend anhand eines in der Zeichnung dargestellten Ausführungsbeispiels erläutert. In der Zeichnung zeigt

die Figur 1     eine Schrägansicht von verschiedenen Teilen einer Einrichtung für die Wunddrainage und

die Figur 2     einen Teil einer Saugvorrichtung, teils in Ansicht und teils im Schnitt.

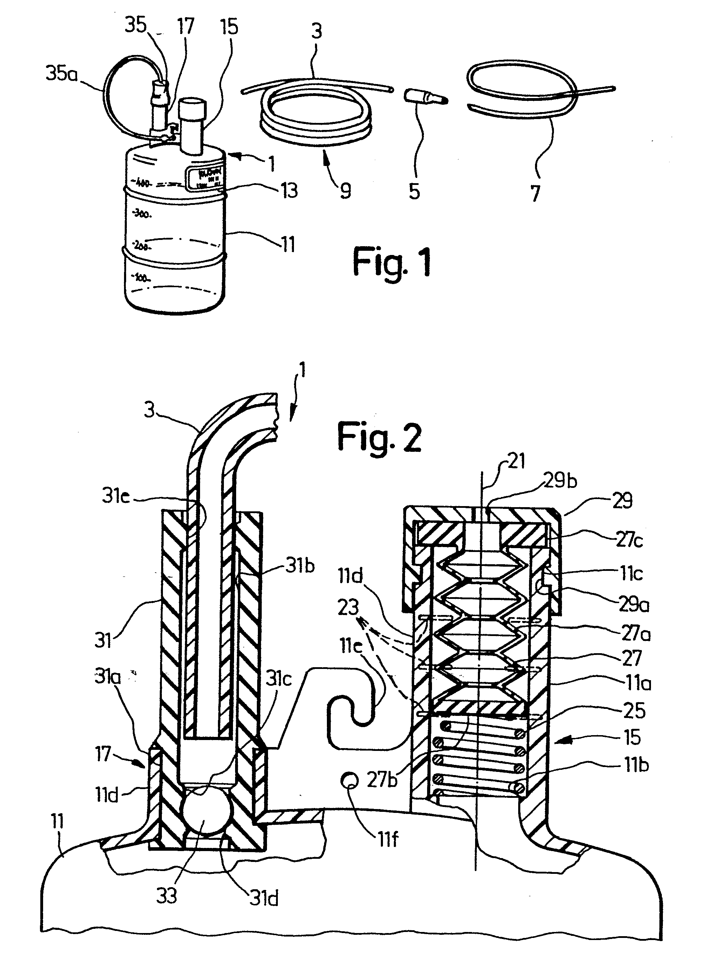

Zu der in der Figur 1 dargestellten Einrichtung für die Wunddrainage gehört eine Saugvorrichtung 1 und ein flexibler Verbindungsschlauch 3, ein Kupplungsstück 5 und ein Saugschlauch 7, der mit einer Anzahl über einen Teil seiner Länge verteilter Sauglöcher versehen ist. Die beiden Schläuche 3, 7 und das Kupplungsstück 5 dienen zur Bildung einer Drainageleitung 9. Die Saugvorrichtung 1 weist als Hauptbestandteil ein formfestes Sauggefäss 11 auf, dessen Wandung einen Boden, einen Mantel und eine gewölbte Decke aufweist. Die Wandung des Sauggefässes 11 ist durchsichtig und der Mantel ist mit einer eingeprägten Skala zur Anzeige des Volumens des im Sauggefäss vorhandenen Wundsekrets versehen. Im übrigen ist die Wandung des Sauggefässes 11 vorzugsweise im allgemeinen farblos, wobei aber der Mantel mit einer farbigen, aufgedruckten Bezeichnung 13, nämlich einer Typenbezeichnung, versehen sein kann. Die Saugvorrichtung 1 weist ferner eine Vakuum-Anzeigevorrichtung 15 zur Erfassung und Anzeige des im Sauggefäss-Innenraum vorhandenen Unterdruckes, d.h. der Druckdifferenz zwischen der Umgebung und dem Sauggefäss-Innenraum, und einen Anschluss 17 zum Anschliessen des Verbindungsschlauchs 3 auf.

Nun soll anhand der Figur 2 die Ausbildung der Vakuum-Anzeigevorrichtung 15 und des Anschlusses 17 näher erläutert werden. Zur Bildung der Vakuum-Anzeigevorrichtung 15 ist an der Decke des Sauggefässes ein von dieser weg nach oben ragender, formfester, starr mit der Sauggefäss-Decke verbundener Stutzen 11a angeformt, der im allgemeinen rotationssymmetrisch zu einer Achse 21 ist und an seinem freien Ende eine ringförmige, mit der Achse 21 einen rechten Winkels bildende Endfläche aufweist. Der Stutzen 11a besitzt eine entlang seiner Achse 21 verlaufende, durchgehende Öffnung. Diese ist in der Nähe der Verbindungsstelle des Stutzens 11a mit der Decke des Sauggefässes 11 mit einer Verengung versehen, die eine ringförmige Auflagefläche 11b bildet. Der Stutzen 11a ist auf seiner Aussenseite in der Nähe seines oberen Endes mit einer ringförmigen Rast-Nut 11c versehen.

In der Öffnung des Stutzens 11a ist eine Feder 25, nämlich eine Schrauben-Druckfeder, eingesetzt, die aus einem metallischen Draht besteht und deren Hauptteil mit geringem, radialem Spiel in der Öffnung geführt ist, wobei der Windungsdurchmesser des oberen Endabschnitts der Feder etwas kleiner sein kann als derjenige des Feder-Hauptteils. Das untere Ende der Feder 25 liegt auf der Auflagefläche 11b auf und ist also dort druckfest gehalten.

Ein einstückiges, elastisches, nämlich gummielastisches Mess- und Anzeigeorgan 27 weist einen länglichen Balg 27a auf, der mit mindestens einer um die Achse 21 herumlaufenden Falte und vorzugsweise mit mehreren in axialer Richtung gegeneinander versetzten Falten versehen ist. Der Innenraum des Balgs 27a ist an seinem unteren, dem Sauggefäss-Innenraum zugewandten Ende durch eine Stirnwand dicht abgeschlossen, die einen Anzeigeteil 27b zum Anzeigen der genannten Druckdifferenz bildet. Der Anzeigeteil 27b ist auf einander abgewandten Seiten mit Flächen versehen, die mit der Achse 21 einen Winkel bilden, wobei vorzugsweise mindestens die untere dieser Flächen eben und rechtwinklig zur Achse 21 ist. Das Mess- und Anzeigeorgan 27 ist am oberen Ende des Balgs 27a mit einem von diesem weg radial nach aussen ragenden Kragen 27c versehen, der auf der radialen Endfläche des Stutzens 11a aufliegt.

Eine kappenförmige, auf den Stutzen 11a aufgeklipste Klemmhülse 29 besitzt einen hohlzylindrischen Mantel, der auf seiner Innenseite mit einer ringförmigen Rast-Rippe 29a versehen ist, die in die Rast-Nut 11c des Stutzens 11a eingreift und die Klemmhülse an diesem festhält. Die Klemmhülse 29 weist an ihrem oberen Ende eine Deckwand auf, die den Kragen 27c des Mess- und Anzeigeorgans 27 gegen die radiale Endfläche des Stutzens 11a drückt und dicht am Stutzen 11a festklemmt, so dass also der Anzeigeteil 27b durch den Balg 27a und den Kragen 27c dicht mit dem Stutzen 11a verbunden ist. Der Kragen 27c schliesst also den Stutzen 11a oder genauer gesagt, dessen zwischen der Innenfläche der Stutzen-Wandung und der Aussenfläche des Balgs 27a vorhandenen Innenraumbereich dicht gegen die Umgebung des Sauggefässes ab. Der Balg 27a ist am kragenseitigen Ende offen und die Deckwand der Klemmhülse 29 besitzt im Zentrum eine Öffnung 29b, die den Innenraum des Balgs 27a fluidmässig mit der Umgebung verbindet. Die obere, dem Innenraum des Balgs 28a zugewandte Fläche des Anzeigeteils 27b grenzt also an einen fluidmässig mit der Umgebung verbundenen Raumbereich an. Die untere Fläche des Anzeigeteils 27b grenzt an einen fluidmässig mit dem Innenraum des Sauggefässes verbundenen Raumbereich des Innenraums des Stutzens 11a an. Die Feder 25 greift an der unteren Fläche des Anzeigeteils 27b an und übt auf diesen eine vom Innenraum des Sauggefässes 11 weg nach oben gerichtete Kraft aus. Nun ist ja nicht nur die Feder 25, sondern auch der Balg 27a federnd. Der Elastizitätsmodul des die Feder 25 bildenden, metallischen Materials ist jedoch wesentlich grösser als derjenige des den Balg 27a und die restlichen Abschnitte des Mess- und Anzeigeorgans 27 bildenden, gummielastischen Materials. Des weiteren sind die Feder 25 und der Balg 27a vorzugsweise derart ausgebildet und bemessen, dass die Federkonstante der Feder 25 grösser ist als diejenige des Balgs 27a. Im übrigen ist auch der Elastizitätsmodul des das Sauggefäss 11 und den Stutzen 11a bildenden Materials wesentlich grösser als derjenige des das Mess- und Anzeigeorgan 27 bildenden Materials.

Der Stutzen 11a ist mit einer Skala versehen, die durch beispielsweise drei entlang der Achse 21 gegeneinander versetzte, strichförmige Markierungen 23 und diesen zugeordneten, nicht dargestellten, etwa bei Unterbrechungen der strichförmigen Markierungen angeordneten Grössen- oder Massangaben gebildet ist, die die Grösse des im Sauggefäss-Innenraum vorhandenen Unterdrucks charakterisieren. Der untersten Markierung 23 ist beispielsweise die Angabe "max", der mittleren Markierung 23 die Angabe "1/2" und der obersten Markierung 23 die Angabe "min" zugeordnet, wobei die Markierungen und Angaben beispielsweise durch Aufdrucke oder durch eingefärbte Kerben gebildet sind. Die Wandung des Stutzens 11a ist mindestens teilweise und vorzugsweise abgesehen von den Markierungen 23 und Grössen- oder Massangaben vollständig durchsichtig und farblos. Das Mess- und Anzeigeorgan 27 besteht aus undurchsichtigem Material und besitzt vorzugsweise eine Farbe, die es durch die Wandung des Stutzens 11a hindurch gut sichtbar macht. Die Klemmhülse 29 ist beispielsweise undurchsichtig.

Für die Bildung des Anschlusses 17 ist an der Decke des Sauggefässes 11 ein nach oben von diesem wegragender, kurzer, im allgemeinen zylindrischer, formfester Stutzen 11d angeformt. Eine elastische, deformierbare, nämlich gummielastische, längliche, im allgemeinen zylindrische Hülse 31 weist beim einen Endabschnitt aussen eine Verdickung auf, die durch eine ringförmige Nut 31a in zwei Teile unterteilt ist. Die Verdickung und die Nut 31a bilden zusammen Rastmittel, so dass das sich in der Figur 2 unten befindende Ende der Hülse 31 beim Zusammenbauen der Saugvorrichtung unter einer vorübergehenden Kompression durch den Stutzen 11d hindurch in den Innenraum des Sauggefässes 11 hineingeschoben werden kann und die Hülse 31 beim Erreichen der in der Figur 2 dargestellten Stellung im Stutzen 11d einrastet und dann derart unter elastischer Vorspannung steht und so fest in diesem sitzt, dass der Verbindungsschlauch 3 in sie eingeführt und wieder aus ihr herausgezogen werden kann, ohne dass sie vom Stutzen 11d getrennt wird. Im übrigen ist der untere Nutrand entsprechend der Wölbung der Decke des Sauggefässes geformt, so dass die Hülse 31 durch den nicht zur Hülsenachse rotationssymmetrischen Nutrand auch mindestens einigermassen gegen Drehungen um die Hülsenachse gesichert ist. Der Durchgang des Anschlusses 17 ist mindestens zum Teil und im vorliegenden Fall vollständig durch den in der Längsrichtung der Hülse 31 verlaufenden Durchgang 31b der Hülse 31 gebildet. Der Durchgang 31b ist zumindest im allgemeinen bezüglich der Hülsenachse rotationssymmetrisch und zum grössten Teil durch einen zylindrischen Hauptabschnitt gebildet, der den weitesten Durchgangsabschnitt bildet und an dessen unteres Ende ein etwas engerer zylindrischer Abschnitt 31c anschliesst, der den stutzenseitigen Endabschnitt des Durchgangs 31b bildet. Dieser engere Abschnitt 31c ist durch eine noch engere, sich in der Nähe der unteren Mündung des Durchgangs 31b befindende Verengung 31d in zwei Teile unterteilt, wobei die Verengung 31d durch einen ringförmigen, im Schnitt gewölbt nach innen vorstehenden Wulst gebildet ist. Ferner ist der Durchgang 31b auch in der Nähe seiner oberen Mündung mit einer im allgemeinen zylindrischen Verengung 31e versehen, die den erwähnten zylindrischen Hauptabschnitt ebenfalls in zwei Teile unterteilt, von denen der untere, sich von der Verengung 31e bis zum engeren Abschnitt 31c erstreckende länger ist und sich mindestens über die halbe Länge des ganzen Durchgangs 31b erstreckt.

Ein durch eine formfeste Kugel gebildetes Verschlusselement 33 sitzt im vom Stutzen 11d umschlossenen Bereich auf der oberen Seite der Verengung 31e im engeren Durchgangsabschnitt 31c und wird dort durch die Hülse 31 derart eingeklemmt und gehalten, dass es den Durchgang 31b dicht, und zwar gas- und vakuumdicht, abschliesst, wobei das Verschlusselement vorzugsweise an einer Fläche der Verengung 31d anliegt.

In der Figur 2 ist auch noch der eine in die Hülse gesteckte Endabschnitt des Verbindungsschlauchs 3 ersichtlich. Der Verbindungsschlauch 3 ist flexibel, wobei aber der ganze Verbindungsschlauch oder mindestens dessen erwähnter Endabschnitt so steif ist, dass er in den Durchgang 31b hineinstossbar ist. Der Verbindungsschlauch 3 oder mindestens sein in den Durchgang 31b steckbarer Endabschnitt ist rotationssymmetrisch zu seiner Längsachse, nämlich zylindrisch. Der Durchmesser des zylindrischen Hauptabschnitts des Durchgangs 31b ist grösser als der Durchmesser der Aussenfläche des genannten Endabschnitts des Verbindungsschlauchs 3. Die Durchmesser des engeren Abschnitts 31c und der beiden Verengungen 31d, 31e des Durchgangs 31b sind dagegen kleiner als der Durchmesser der Aussenfläche des genannten Endabschnitts des Verbindungsschlauchs 3. Diese Bemessung der Durchmesser gewährleistet, dass der Verbindungsschlauch 3 relativ leicht in die Hülse 31 eingeführt werden kann, aber trotzdem bereits vor dem Erreichen des Verschlusselements 33, oder zumindest bevor er dieses ganz aus der Hülse 31 herausgestossen hat, vakuumdicht mit der Hülse 31 verbunden wird und dann beim Erreichen der vorgesehenen Endstellung vakuumdicht und ausreichend fest in dieser sitzt. Die Abdichtung erfolgt dabei vor allem beim engeren, zylindrischen Abschnitt 31c und, falls der Verbindungsschlauch durch die Verengung 31d hindurch gesteckt wird, auch bei dieser. Die Verengung 31e trägt natürlich auch zur Abdichtung bei, dient aber vor allem dazu, den Verbindungsschlauch beim Einschieben in die Hülse 31 zu zentrieren und zu führen und ihn nachher im eingesetzten Zustand in der Nähe des oberen, dem Sauggefäss-Innenraum abgewandten Endes der Hülse 31 koaxial zu halten.

An die Decke des Sauggefässes 11 ist zwischen den beiden Stutzen 11a, 11d ein Steg mit einem Haken 11e und einem Durchgangsloch 11f angeformt. Ein nur in der Figur 1 dargestelltes Verschlussorgan 35 besitzt eine Kappe und einen mindestens teilweise von deren zylindrischem Mantel umschlossenen und zum letzteren koaxialen Zapfen. Das Verschlussorgan 35 ist lösbar mit dem dem Sauggefäss 11 abgewandten Ende der Hülse 31 des Anschlusses 17 verbindbar, nämlich auf die Hülse 31 aufsteckbar. Wenn das Verschlussorgan 35 auf die Hülse 31 aufgesteckt ist, ragt sein Zapfen in die Längsöffnung der Hülse 31 hinein und die Kappe 35 bedeckt und umgreift das Hülsenende, so dass das Verschlussorgan 35 den Durchgang 31b mindestens flüssigkeitsdicht und vorzugsweise gasdicht schliesst. Das Verschlussorgan 35 ist auf der dem Zapfen abgewandten Seite der Kappe mit einem schwanz- und saitenartigen, flexiblen Aufhänger 35a versehen, der an seinem der Kappe abgewandten Ende im Loch 11f befestigt ist.

Das Sauggefäss 11 besteht zusammen mit den an seiner Decke angeformten Stutzen 11a, 11d und dem zwischen diesen vorhandenen Steg aus einem einstückigen Körper aus durchsichtigem, farblosem Kunststoff, beispielsweise aus dem unter dem Handelsnamen KODAR granulatförmig von der Eastman Chemical Products Inc., Kingsport, Tennesse, gelieferten Copolymer. Das Mess- und Anzeigeorgan 27 und die Hülse 31 bestehen aus synthetischem und/oder natürlichem Gummi, beispielsweise Nitrilkautschuk. Die Klemmhülse 29 besteht aus einem Kunststoff, beispielsweise einem Thermoplast. Dieser ist einerseits ausreichend federnd, damit die Klemmhülse 29 auf den Stutzen

11a aufgeklipst werden kann, und andererseits ausreichend fest, um den Kragen 27c dicht festzuklemmen. Das Verschlussorgan 35 und der mit diesem zusammen aus einem einstückigen Körper gebildete Aufhänger 35a, die Schläuche 3, 7 und das Kupplungsstück 5 bestehen ebenfalls aus einem Kunststoff, beispielsweise einem Thermoplast. Die Feder 25 und das Verschlusselement 33 bestehen aus Metall, beispielsweise rostfreiem Stahl. Die die verschiedenen Teile der Saugvorrichtung 1 bildenden Materialien sind alle beständig gegen Bestrahlung mit Gammastrahlen, wobei insbesondere das Sauggefäss 11 und dessen Stutzen 11a bei einer solchen Bestrahlung durchsichtig und farblos bleiben.

Für die Bereitstellung von Einrichtungen der in der Figur 1 dargestellten Art werden zuerst die verschiedenen Einzelteile hergestellt. Dabei wird zur Bildung des Sauggefässes 11 zuerst ein Schlauch extrudiert und dieser dann stückweise mit zwei je einen Durchgang aufweisenden Blasdornen in eine zwei trennbare, schalenförmige Teile aufweisende Form hineingeblasen, wobei das die beiden Blasdorne unschliessende Kunststoffmaterial die beiden Stutzen 11a, 11d bildet. Danach können noch die Bezeichnung 13, die Markierungen 23 und die den letzteren zugeordneten Grössen- oder Massangaben aufgedruckt werden. Nach der Herstellung des Sauggefässes 11 und der übrigen Einzelteile werden die Feder 25 und das Mess- und Anzeigeorgan 27 in den Stutzen 11a eingesetzt, die Klemmhülse 29 auf den Stutzen 11a aufgeklipst, die Hülse 31 in den Stutzen 11d eingesetzt und der Schwanz 35a der Verschlusskappe 35 am Sauggefäss befestigt. Danach wird im Innenraum des Sauggefässes durch Absaugen von Luft ein Unterdruck erzeugt. Anschliessend wird das Verschlusselement 33 in die Hülse 31 eingesetzt, so dass es in die in der Figur 2 dargestellte Lage gelangt, in der es an der als Anschlag dienende Verengung 31d ansteht. Dabei wird beim Einsetzen des Verschlusselements 37 der Unterdruck im Innenraum des Sauggefässes aufrechterhalten. Daraufhin werden entweder die Verschlusskappe 35 auf die Hülse 31 aufgesetzt oder ein Verbindungsschlauch 3 bis etwa zu der in der Figur 2 dargestellten Tiefe in die Hülse 31 eingesetzt und die so gebildete Saugvorrichtung 1, allenfalls mitsamt dem in die Hülse 31 gesteckten Verbindungsschlauch 3, luftdicht in einem Kunststoffbeutel verpackt. Nun wird die Saugvorrichtung noch durch Bestrahlen mit Gammastrahlen steril gemacht. Falls der Verbindungsschlauch 3 nicht zusammen mit der Saugvorrichtung verpackt und steril gemacht wurde, wird er separat zusammen mit dem Kupplungsstück 9 und dem Drainageschlauch 7 verpackt, wobei natürlich die Schläuche 3, 7 und das Kupplungsstück 9 auf jeden Fall auch steril gemacht werden. Auf diese Weise können die Einrichtungen für die Wunddrainage sehr kostengünstig hergestellt und steril gemacht werden.

Im übrigen können Saugvorrichtungen mit verschiedener Saugkraft, d.h. mit verschiedenen Nenn- oder Anfangs-Unterdrücken hergestellt werden. Man kann beispielsweise drei Typen von Saugvorrichtungen vorsehen, deren Anfangs-Unterdrücke, d.h. Anfangs-Druckdifferenzen gegenüber dem Umgebungs-Luftdruck, ungefähr 90, 60 bzw. 30 Kilopascal betragen. Die durch einen Aufdruck gebildete Bezeichnung 13 kann dann beispielsweise eine Angabe des Nenn- oder Anfangs-Unterdrucks und je nach Typ eine der Angaben "Vacuum stark", "Vacuum mittel" oder "Vacuum schwach" enthalten, wobei die Bezeichnungen für die verschiedenen Typen verschiedenfarbig sein können. Die Sauggefässe mit grossen AnfangsUnterdrücken können dann beispielsweise für die Wunddrainage von Operationswunden in unempfindlichen Körperbereichen erwachsener Personen verwendet werden. Die Sauggefässe mit mittleren oder kleinen Anfangs-Unterdrücken können für die Wunddrainage in empfindlichen Körperbereichen, wie beispielsweise im Hirnbereich, und/oder bei Operationen kleiner Kinder verwendet werden. Die Sauggefässe 11 und die an ihm angeformten Stutzen 11a, 11d können bei allen Typen die gleichen Formen und Abmessungen aufweisen. Die Mess- und Anzeigeorgane 27 können ebenfalls bei allen Typen die gleichen Formen und Abmessungen aufweisen, wobei aber die Farben für die verschiedenen Typen verschieden gemacht werden können, so dass bei jedem Typ die Farbe des Mess- und Anzeigeorgans mit derjenigen der Bezeichnung 13 auf dem Sauggefäss übereinstimmt. Die Federn 25 können für die verschiedenen Typen der Saugvorrichtung unterschiedlich ausgebildet werden, so dass sie verschiedene Federkonstanten besitzen und bei der in der Figur 2 gezeichneten Stellung des Anzeigeteils 27b verschiedene Kräfte erzeugen. Dadurch kann erreicht werden, dass sich der Anzeigeteil 27b des Mess- und Anzeigeorgans 27 bei jedem der drei verschiedenen Saugvorrichtungstypen beim Vorhandensein des vorgesehenen Anfangs-Unterdrucks bei der untersten Markierung 23 befindet und sich beim Absinken des Unterdrucks auf Null bis zur obersten Markierung 23 oder bis in den vor der undurchsichtigen Klemmhülse 29 bedeckten Abschnitt des Stutzens 11a bewegt. Der Anzeigeteil 27b wird also bei jedem der drei Saugvorrichtungstypen beim Absinken des Unterdrucks vom vorgesehenen Anfangswert auf Null, d.h. beim Ansteigen des absoluten Drucks im Sauggefäss auf den Umgebungs-Luftdruck, um eine mindestens annähernd gleich lange Strecke verschoben. Die Klemmhülsen 29 und die den Anschluss 17 bildenden Teile können bei allen vorgesehenen Saugvorrichtungstypen gleich ausgebildet werden. Wenn also verschiedene Saugvorrichtungstypen mit verschiedenen Anfangs-Unterdrücken bereitgestellt werden sollen, braucht man, abgesehen von den verschiedenen Bezeichnungen 13 und allenfalls verschiedenfarbig ausgebildeten Mess- und Anzeigeorganen 27, lediglich verschiedene Federn herzustellen und die Sauggefässe dann unterschiedlich stark zu evakuieren. Das ermöglicht, kostengünstig verschiedene Typen von Saugvorrichtungen bereitzustellen.

Wenn nun bei oder nach einer Operation eine Wunddrainage vorgenommen werden soll, wird zuerst der Saugschlauch 7 mit einer Nadel in die Wunde eingezogen und mit dem Kupplungsstück 9 mit dem Verbindungsschlauch 3 verbunden. Dieser wird nun, falls er nicht bereits in der Hülse 31 steckt, manuell in diese eingesteckt. Wenn man nun das sich in der Hülse 31 befindende Ende des Verbindungsschlauchs 3 bis ungefähr zum unteren Ende der Hülse 31, beispielsweise bis mindestens zur Verengung 31d oder durch den ganzen Durchgang 31b hindurch, gegen den Innenraum des Sauggefässes 11 in die Hülse hinein drückt, stösst der Verbindungsschlauch 3 das in der Hülse 31 eingeklemmte Verschlusselement 33 aus der Hülse 31 heraus in den Innenraum des Sauggefässes 11 hinein. Der Durchgang 31b enthält dann zwar mindestens in einem Teilbereich seiner Länge einen Abschnitt des Verbindungsschlauchs 3. Da dieser ja ebenfalls einen Durchgang besitzt, wird jedoch der Durchgang 31b beim Herausstossen des Verschlusselements 33 durchströmbar gemacht und strömungsmässig irreversibel freigegeben und also der Innenraum des Sauggefässes 11 fluidmässig dicht mit der Drainageleitung 9 verbunden. Da das Sauggefäss 11 durchsichtig ist, kann man ohne weiteres sehen, wann das Verschlusselement 33 in den Innenraum des Sauggefässes hineinfällt und der Durchgang 31b freigegeben wird. Die Drainageleitung 9 kann also rasch und einfach zusammengesetzt und vakuumdicht sowie steril mit dem Anschluss 17 der Saugvorrichtung 1 verbunden werden, wobei eine Person in sehr einfacher Weise den vorher mit dem Verschlusselement 33 dicht verschlossenen bzw. versperrten Durchgang des Anschlusses 17 zuverlässig durchgängig machen bzw. freigeben und diese Freigabe visuell feststellen kann.

Der Aufhänger 35a kann bei der Benutzung der Saugvorrichtung beispielsweise schlaufenförmig um eine Stange des Bettgestells des Patienten oder um einen am Bettgestell, einem sonstigen Gestell oder einer Wand befestigten Haken oder dergleichen gelegt werden, wonach das mit der Verschlusskappe zusammenhängende Ende des Aufhängers 35a in den Haken 11e eingehängt werden kann. Auf diese Weise kann die Saugvorrichtung 1 in der Nähe des Patienten aufgehängt werden.

Nach dem Herausdrücken des Verschlusselements 33 aus der Hülse 31 saugt die Saugvorrichtung 1 infolge des im Innenraum des Sauggefässes 11 herrschenden Unterdruckes Wundsekret aus der Wunde in das Sauggefäss 11 hinein. Dabei nimmt der Unterdruck, d.h. die Druckdifferenz zwischen dem in der Umgebung des Sauggefässes 11 herrschenden Luftdruck und dem Druck im Innenraum des Sauggefässes 1, ab. Die Grösse des Unterdruckes, d.h. der genannten Druckdifferenz, wird von der Volumen-Anzeigevorrichtung 13 erfasst und angezeigt. Beim bei der Herstellung der Saugvorrichtung 1 im Sauggefäss-Innenraum erzeugten, Nenn- oder Anfangs-Unterdruck befindet sich der Anzeigeteil 27b des Mess- und Anzeigeorgans 27 in der in der Figur 2 gezeichneten Stellung bei der beispielsweise mit "max" bezeichneten Markierung. Wenn nun der Unterdruck im Sauggefäss abnimmt, drückt die Feder 25 den Anzeigeteil 27b sukzessive immer weiter vom Innenraum des Sauggefässes 11 weg nach oben. Wenn der Druck im Sauggefäss auf die Grösse des Luftdruckes in der Umgebung des Sauggefässes angestiegen ist, gelangt der die untere Begrenzung des Mess- und Anzeigeorgans 27 bildende Anzeigeteil 27b bis zur beispielsweise mit "min" bezeichneten Markierung oder bis in den von der Klemmhülse 29 bedeckten Abschnitt des Stutzens 11a. Eine den Patienten betreuende Pflegeperson kann nun aus der Stellung des Anzeigeteils 27b mühelos die Grösse des Unterdrucks ablesen und insbesondere erkennen, wann dieser auf Null oder einen anderen vorgesehenen Minimalwert abgesunken ist.

Wenn nun der Unterdruck auf einen vorgesehene Minimalwert abgesunken ist, kann die Pflegeperson das Sauggefäss 11 durch ein neues Sauggefäss ersetzen. Der Anschluss 15 des alten, Wundsekret enthaltenden, für den einmaligen Gebrauch vorgesehenen Sauggefässes 11 kann dabei durch Aufsetzen der Verschlusskappe 35 dicht verschlossen werden. Die Verschlusskappe 35 dient also sowohl zum Verschliessen des Anschlusses 15 als auch zusammen mit dem Aufhänger 35a zum Aufhängen des Sauggefässes 11 an einer Stange, einem Haken oder dergleichen.

Bei der Messung und Anzeige des Unterdruckes wird der angezeigte Wert, d.h. die Stellung des Anzeigeteils 27b im vor allem durch das Kräftegleichgewicht zwischen der infolge der Druckdifferenz zwischen Druck im Sauggefäss-Innenraum und der Umgebung auf den Anzeigeteil 27b ausgeübten Gasdruckkraft und der von der Feder 25 auf den Anzeigeteil 27b ausgeübten Federkraft bestimmt. Da der Balg 27a elastisch deformierbar ist, erzeugt er ebenfalls eine von seiner jeweiligen Länge abhängige Kraft, die aber zumindest bei den in der Figur 2 gezeichneten Stellungen und Formen der Feder 25 und des Mess- und Anzeigeorgans 27 sowie mindestens auch bei allen anderen beim Vorhandensein einer Druckdifferenz zwischen der Umgebung und dem Sauggefäss-Innenraum von der Feder und dem Anzeigeorgan eingenommenen Stellungen und Formen im Vergleich zu der von der Feder auf das Mess- und Anzeigeorgan 27 ausgeübten Kraft klein ist. Wenn nach der Benutzung der Saugvorrichtung keine Druckdifferenz zwischen der Umgebung und dem Sauggefäss-Innenraum mehr vorhanden ist, drückt die nun ihre längste Form aufweisende Feder 25 den Balg 27a derart zusammen, dass dessen durch Falten getrennte bzw. gebildete Abschnitte mindestens annähernd aneinander anliegen und ein praktisch kompaktes Paket bilden. Die von der Feder in diesem End-Zustand noch auf den Anzeigeteil 27b ausgeübte Kraft wird dann durch den Balg 27a und/oder durch andere Teile des Mess- und Anzeigeorgans 27 und/oder die Klemmhülse 29 sowie den Stutzen 11a aufgenommen und kompensiert.

Bei der Herstellung der Saugvorrichtungen kann ohne weiteres eine grosse Anzahl Federn 25 hergestellt werden, deren Abmessungen und Federkonstanten genügend genau den vorgesehenen Grössen entsprechen, um eine ausreichende Genauigkeit bei der Ermittlung und Anzeige des Unterdrucks zu erzielen.

Die Einrichtung kann in verschiedener Hinsicht modifiziert werden.

Beispielsweise könnte die Bezeichnung 13 statt durch einen unmittelbar auf das Sauggefäss 11 aufgedruckten Aufdruck auch durch eine auf das Sauggefäss aufgeklebte Etikette gebildet sein.

Anstelle des einstückigen Mess- und Anzeigeorgans 27 könnte ein formfester, die Funktion des Anzeigeteils 27b übernehmender Zapfen vorgesehen werden, der mit einem separaten, flexiblen Balg in einem dem Stutzen 11a entsprechenden Stutzen gehalten und vorzugsweise hohl und am oberen Ende durch eine Stirnwand abgeschlossen ist. Der eine, dem Sauggefäss-Innenraum zugewandte Balg-Endabschnitt könnte dann dicht und fest auf die Aussenflächen des Stutzens und der andere Balg-Endabschnitt dicht und fest auf einen aus dem Stutzen herausragenden Teil des Zapfens aufgezogen sein. Der Balg könnte zwischen den beiden genannten Endabschnitten eine entlang seinem Umfang verlaufende, im Schnitt S-förmige Falte bilden, so dass der Balg den Zapfen dicht und axial verschiebbar mit dem Stutzen verbindet. Die der Feder 25 entsprechende Feder greift an diesem Zapfen an und kann, wenn dieser hohl ist, bis zu seiner Stirnwand in ihn hineinragen. Der den Anzeigeteil bildende Zapfen wird dann durch die Feder abhängig von der momentanen Grösse des Unterdruckes im Sauggefäss mehr oder weniger weit nach oben aus dem Stutzen herausgedrückt. Im übrigen kann der Zapfen bzw. der auf ihn aufgezogene Balgendabschnitt mit in axialer Richtung gegeneinander versetzten Markierungen versehen sein, so dass aus der vom Zapfen bezüglich des Stutzens eingenommenen Schiebestellung mühelos die Grösse des Unterdrucks ersehen werden kann.

Die Feder 25 der in den Figuren 1 und 2 dargestellten Saugvorrichtung sowie die Feder der im vorgängigen Absatz beschriebenen Variante der Saugvorrichtung könnten statt aus einem metallischen Draht auch aus elastischem Kunststoff bestehen. Die von der Feder auf den Anzeigeteil ausgeübte Kraft soll jedoch unabhängig vom Federmaterial mindestens in den von ihr und vom Balg beim Vorhandensein einer Druckdifferenz zwischen der Umgebung und dem Sauggefäss-Innenraum eingenommenen Stellungen und Formen grösser, und vorzugsweise wesentlich grösser, sein als die durch die Deformation des Balgs von diesem erzeugte Kraft.

Das kugelförmige Verschlusselement 33 könnte beispielsweise durch ein konisches oder teils zylindrisches und teils konisches Verschlusselement ersetzt werden, das mit dem Verbindungsschlauch der Drainageleitung aus der der Hülse 31 entsprechenden, gummielastischen Hülse herausgedrückt werden kann. Das Verschlusselement könnte zudem statt durch einen aus einer Hülse herausstossbaren Körper durch eine Membran gebildet sein, die beispielweise am unteren Ende der der Hülse 31 entsprechenden Hülse angeordnet ist, den Durchgang des Anschlusses vor der Benutzung des Sauggefässes dicht versperrt und mit dem Verbindungsschlauch der Drainageleitung zum Freigeben des Durchgangs durchstossen werden kann. Diese Membran könnte dabei zusammen mit der Hülse, in die der Verbindungsschlauch gesteckt wird, aus einem einstückigen Körper bestehen. Im übrigen könnte die Hülse abgesehen von der zusätzlich vorhandenen Membran ähnlich ausgebildet sein wie die Hülse 31, wobei aber eventuell die Verjüngung 31d weggelassen werden könnte. Bei einer derartigen Ausbildung des Anschlusses wäre die die Membran aufweisende Hülse bei der Fabrikation und Bereitstellung der Saugvorrichtung selbstverständlich erst nach der Evakuierung des Sauggefässes in dessen zur Bildung des Anschlusses dienenden Stutzen einzusetzen.

Der zum Einstecken in die Hülse 31 bestimmte Endabschnitt der Drainageleitung könnte statt durch die zy-

lindrischen Endabschnitt des Verbindungsschlauchs 3 durch ein Leitungsstück gebildet sein, von dem sich mindestens ein Teil zu seinem freien Ende hin verjüngt und konisch ist. Des weitern wäre es sogar möglich, den Durchgang des Sauggefäss-Anschlusses und den in diesen steckbaren Endabschnitt der Drainageleitung im Querschnitt nicht rund, sondern beispielsweise viereckig auszubilden.

Die in den beiden Figuren der Zeichnung dargestellte Saugvorrichtung 1 ist für den einmaligen Gebrauch vorgesehen. Die Saugvorrichtung könnte nun auch dahingehend modifiziert werden, dass sie ganz oder teilweise mehrmals verwendbar ist. Beispielsweise könnte man das Sauggefäss 11 durch ein Sauggefäss ersetzen, das oben eine mit einem lösbar befestigten Verschlussteil verschliessbare Öffnung besitzt. Das Sauggefäss könnte dann aus einem für die Wiederverwendung des Sauggefässes durch Hitze, beispielsweise mit heissem Wasserdampf, sterilisierbaren Material, wie etwa einem mineralischen Glas, bestehen. Der Verschlussteil könnte in diesem Fall mit einer im Prinzip gemäss der Vakuum-Anzeigevorrichtung 15 ausgebildeten Vakuum-Anzeigevorrichtung und einer im Prinzip dem Anschluss 17 entsprechenden Anschluss versehen sein. Falls der Verschlussteil einen gummielastischen Stopfen aufweist oder im wesentlichen aus einem solchen gebildet ist, könnte der dem Stutzen 11a entsprechende Stutzen durch einen separaten, aus einem starren Material bestehenden, am Stopfen befestigten Teil und die der Hülse 31 entsprechende Hülse durch einen am Stopfen angeformten, d.h. mit diesem zusammen aus einem einstückigen Körper bestehenden Fortsatz gebildet sein. Der Verschlussteil, die Vakuum-Anzeigevorrichtung und der Anschluss könnten dann beispielsweise nur für den einmaligen Gebrauch vorgesehen sein, wobei es aber auch möglich wäre, den Verschlussteil und/oder mindestens gewisse Teile der Vakuum-Anzeigevorrichtung und des Anschlusses für den mehrmaligen Gebrauch auszubilden und aus durch Hitze sterilisierbarem Material herzustellen.

PATENTANSPRÜCHE

1. Saugvorrichtung für die Wunddrainage, mit einem Sauggefäss (11) und einer Vakuum-Anzeigevorrichtung (15), die einen Stutzen (11a) und einen mit einem deformierbaren, mindestens eine Falte aufweisenden Balg (27a) entlang der Achse (21) des Stutzens (11a) bewegbar mit diesem verbundenen Anzeigeteil (27b) aufweist, der durch eine infolge einer Druckdifferenz zwischen der Umgebung und dem Sauggefäss-Innenraum auf ihn ausgeübte Fluiddruckkraft entgegen einer durch eine elastische Deformation erzeugten Rückstellkraft verschiebbar ist, dadurch gekennzeichnet, dass eine Feder (25) vorhanden ist, die am Anzeigeteil (27b) angreift.

2. Saugvorrichtung nach Anspruch 1, dadurch gekennzeichnet, dass die Feder (25) eine Schraubenfeder ist.

3. Saugvorrichtung nach Anspruch 1 oder 2, dadurch gekennzeichnet, dass die Feder (25) mindestens in denjenigen Stellungen, die sie einnimmt, wenn zwischen der Umgebung und dem Sauggefäss-Innenraum eine Druckdifferenz vorhanden ist, eine grössere Kraft erzeugt als der Balg (27a).

4. Saugvorrichtung nach einem der Ansprüche 1 bis 3, dadurch gekennzeichnet, dass der Stutzen (11a) aus einem Material besteht, dessen Elastizitätsmodul grösser als derjenige des den Balg (27) bildenden Materials ist.

5. Saugvorrichtung nach einem der Ansprüche 1 bis 4, dadurch gekennzeichnet, dass sich die Feder (25) und der Anzeigeteil (27b) mindestens teilweise im Innern des Stutzens (11a) befinden.

6. Saugvorrichtung nach einem der Ansprüche 1 bis 5, dadurch gekennzeichnet, dass die Feder (25) eine Druckfeder ist, die den Anzeigeteil (27b) mit einer vom Innenraum des Sauggefässes (11) weggerichteten Kraft beaufschlägt.

7. Saugvorrichtung nach Anspruch 6, dadurch gekennzeichnet, dass der Stutzen (11a) mindestens teilweise und vorzugsweise vollständig aus einem durchsichtigen Material besteht, dass sich der Balg (27a) und der Anzeigeteil (27b) im Innern des Stutzens (11a) befinden, dass der Balg (27a) an seinem dem Innenraum des Sauggefässes abgewandten Ende mit einem von ihm weg nach aussen ragenden Kragen (27c) versehen ist, der auf der ringförmigen, mit der Achse (21) des Stutzens (11a) einen vorzugsweise rechten Winkel bildenden Endfläche des Stutzens (11a) aufliegt und mit einer Klemmhülse (29) dicht am Stutzen (11a) festgeklemmt ist, und dass der Anzeigeteil (27b) den Innenraum des Balgs (27a) an dessen dem Innenraum des Sauggefässes (11) zugewandten Ende dicht abschliesst, wobei der Balg (27a) vorzugsweise mehrere Falten aufweist, der Balg (27a), der Anzeigeteil (27b) und der Kragen (27c) vorzugsweise aus einem einstückigen Körper bestehen und die Klemmhülse (29) vorzugsweise mit Rastmitteln (11c, 29a) am Stutzen (11a) befestigt und beispielsweise undurchsichtig ist.

8. Saugvorrichtung nach einem der Ansprüche 1 bis 7, dadurch gekennzeichnet, dass der Stutzen (11a) oder der Anzeigeteil mit einer Skala versehen ist, die beispielsweise entlang der Achse (21) des Stutzens (11a)

gegeneinander versetze Markierungen (23) und diesen zugeordnete, die Grösse der genannten Druckdifferenz charakterisierende Angaben aufweist.

9. Saugvorrichtung nach einem der Ansprüche 1 bis 8, dadurch gekennzeichnet, dass der Stutzen (11a) an die Wandung des Sauggefässes (11) angeformt ist und mit dieser zusammen aus einem einstückigen Körper besteht.

10. Saugvorrichtung nach den Ansprüchen 7 und 9, dadurch gekennzeichnet, dass das Sauggefäss (11) und der Stutzen (11a) aus einem gegen Bestrahlung mit Gammastrahlen beständigen und bei einer solchen Bestrahlung durchsichtig und farblos bleibenden Material besteht.

Fig. 1

Fig. 2

Europäisches
Patentamt

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | GB-A-2 041 756 (STERIMED GmbH) <br> * Figuren; Seite 2, Zeilen 4-58 * <br><br> --- | 1-6,8 | A 61 M .1/00 |
| A | EP-A-0 036 546 (STERIMED GmbH) <br> * Insgesamt * <br><br> ----- | 1,2 | |

**RECHERCHIERTE SACHGEBIETE (Int. Cl.4)**

A 61 M

Der vorliegende Recherchenbericht wurde für alle Patentansprüche erstellt.

| Recherchenort | Abschlußdatum der Recherche | Prüfer |
|---|---|---|
| DEN HAAG | 15-05-1985 | VEREECKE A. |